(19) Europäisches Patentamt
European Patent Office
Office européen des brevets

(11) **EP 4 206 634 B1**

(12) **EUROPEAN PATENT SPECIFICATION**

(45) Date of publication and mention
of the grant of the patent:
**29.05.2024 Bulletin 2024/22**

(21) Application number: **22187000.9**

(22) Date of filing: **26.07.2022**

(51) International Patent Classification (IPC):
**G01K 7/42** *(2006.01)* **G01K 13/20** *(2021.01)*
**A61B 5/01** *(2006.01)* **A61B 5/00** *(2006.01)*

(52) Cooperative Patent Classification (CPC):
**G01K 13/20; A61B 5/01; A61B 5/681;**
**A61B 5/6824; A61B 5/746; G01K 7/427;**
A61B 2505/07; A61B 2505/09; A61B 2560/0223;
A61B 2560/0247; A61B 2562/0276

(54) **APPARATUS AND METHOD FOR ESTIMATING BODY TEMPERATURE**

GERÄT UND VERFAHREN ZUR SCHÄTZUNG DER KÖRPERTEMPERATUR

APPAREIL ET PROCÉDÉ D'ESTIMATION DE LA TEMPÉRATURE CORPORELLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.12.2021 KR 20210185747**

(43) Date of publication of application:
**05.07.2023 Bulletin 2023/27**

(73) Proprietor: **Samsung Electronics Co., Ltd.
Gyeonggi-do, 16677 (KR)**

(72) Inventors:
• **Lee, So Young**
  **Suwon-si (KR)**
• **Kim, Sang Kyu**
  **Yongin-si (KR)**
• **Lee, Ho Taik**
  **Yongin-si (KR)**
• **Kwon, Bok Soon**
  **Seoul (KR)**

(74) Representative: **Grünecker Patent- und
Rechtsanwälte
PartG mbB
Leopoldstraße 4
80802 München (DE)**

(56) References cited:
**WO-A1-2021/057873 US-A1- 2019 142 280**

**Description**

BACKGROUND

1. Field

Example embodiments of the disclosure relate to an apparatus and a method for estimating core body temperature by using a plurality of sensors.

2. Description of the Related Art

Generally, body temperature is one of four vital signs and has very important clinical significance. A body temperature sensor may be used in various applications, such as checking infections in patients, thermal side effects of medications, or time of ovulation in women, and the like. However, skin temperature and core body temperature may vary depending on external temperature, such that it is difficult to measure the core body temperature by using a portable device such as a wearable device. A general body temperature sensor may be classified into a contact type sensor and a non-contact type sensor. Examples of the contact type sensor may include a sensor for detecting a change in electrical resistance, such as a Resistance Temperature Detector (RTD), a thermistor, etc., a thermocouple for detecting electromotive force, and the like. Further, examples of the non-contact type sensor may include a thermopile, a micro-bolometer, and the like, which measure body temperature by detecting infrared rays radiating from a body surface. A general body temperature measuring technology may be significantly affected by a change in environment factors affecting heat transfer, such as a change in external ambient temperature, humidity, air flow, and the like.
US 2019/142280 A1 relates to a single heat flux sensor arrangement. The arrangement comprises a sensor having a layer of thermally insulating material, an inner temperature measurement means arranged at an inner region of the insulating layer and an outer temperature measurement means arranged at an outer region of the insulating layer; an evaluation unit adapted to receive a temperature input from the inner temperature measurement means and to receive a temperature input from the outer temperature measurement means; and a heating means realized to deliberately raise the temperature of a region of the insulating layer. Further a non-invasive method of measuring a core body temperature of a subject is described.
WO 2021/057873 A1 relates to a human body temperature measurement method, electronic device, and computer-readable storage medium. The human body temperature measurement method is applied to a wrist wearable device that may be a smart watch or smart wristband. When measuring the body temperature of a user, it is possible to measure a first temperature of the forehead of the user by means of a temperature sensor, and, at a closer time, measure a second temperature of the wrist of the user by means of another temperature sensor, and, by means of calculation, display on a display screen a third temperature associated with the first temperature; afterward, it is possible to measure a fourth temperature of the wrist by means of a second temperature sensor, and then, if the fourth temperature is the same as the second temperature, it is possible to use the described relationship to display the third temperature on a display screen.

SUMMARY

The invention is claimed in the independent claims. Preferred embodiments are specified in the dependent claims.
According to a first aspect of the present invention, there is provided an apparatus for estimating body temperature, the apparatus including: a first temperature sensor configured to measure a surface temperature of an object; a second temperature sensor configured to measure a surface temperature of a material, which is positioned between the first temperature sensor and the second temperature sensor; and a processor configured to obtain a heat flux based on the surface temperature of the object and the surface temperature of the material, configured to estimate a wrist temperature of the object based on the obtained heat flux and the surface temperature of the object, and configured to estimate core body temperature of the object based on the estimated wrist temperature of the object and a heat loss coefficient between a core and a wrist.
The material may include air.
At least one of the first temperature sensor or the second temperature sensor may include a thermistor.
According to this first aspect of the present invention, the processor is further configured to obtain the heat flux based on a value obtained by subtracting the surface temperature of the material from the surface temperature of the object.
According to this first aspect of the present invention, the processor is further configured to estimate the wrist temperature of the object based on a resistance value of the material, the heat flux, a predetermined skin heat transfer coefficient, and the surface temperature of the object.

The processor may be further configured to estimate the core body temperature of the object by linearly combining the estimated wrist temperature of the object and the heat loss coefficient between the core and the wrist.

The processor may be further configured to correct the heat loss coefficient between the core and the wrist based on an external temperature at a time of estimating the core body temperature and an external temperature at a calibration time.

The processor may be further configured to obtain a calibration heat flux based on the surface temperature of the object and the surface temperature of the material, which are obtained at a calibration time, and the processor may be further configured to, based on the obtained calibration heat flux being less than or equal to a predetermined reference value, obtain the heat loss coefficient between the core and the wrist.

The processor may be further configured to estimate a calibration wrist temperature of the object based on a resistance value of the material, which is obtained at the calibration time, the calibration heat flux, a predetermined skin heat transfer coefficient, and the surface temperature of the object which is obtained at the calibration time.

The processor may be further configured to obtain the heat loss coefficient between the core and the wrist based on a difference between the estimated calibration wrist temperature of the object and the core body temperature of the object, which is obtained at the calibration time.

The apparatus may further include a display device, and the processor may be further configured to, based on the obtained calibration heat flux exceeding the predetermined reference value, output a text message for inducing a user to reduce the heat flux, to the display device.

According to a second aspect of the present invention, there is provided a method of estimating body temperature, the method including: measuring, by a first temperature sensor, a surface temperature of an object; measuring, by a second temperature sensor, a surface temperature of a material disposed between the first temperature sensor and the second temperature sensor; obtaining a heat flux based on the surface temperature of the object and the surface temperature of the material; estimating a wrist temperature of the object based on the obtained heat flux and the surface temperature of the object; and estimating core body temperature of the object based on the estimated wrist temperature of the object and a heat loss coefficient between a core and a wrist.

According to this second aspect of the present invention, the obtaining the heat flux includes obtaining the heat flux based on a value obtained by subtracting the surface temperature of the material from the surface temperature of the object.

According to this second aspect of the present invention, the estimating the wrist temperature of the object includes estimating the wrist temperature of the object based on a resistance value of the material, the heat flux, a predetermined skin heat transfer coefficient, and the surface temperature of the object.

The estimating the core body temperature of the object may include estimating the core body temperature of the object by linearly combining the estimated wrist temperature of the object and the heat loss coefficient between the core and the wrist.

The method may further include: obtaining a calibration heat flux based on the surface temperature of the object and the surface temperature of the material, which are obtained at a calibration time; and based on the obtained calibration heat flux being less than or equal to a predetermined reference value, obtaining the heat loss coefficient between the core and the wrist.

The obtaining the heat loss coefficient between the core and the wrist may include: estimating a calibration wrist temperature of the object based on a resistance value of the material, which is obtained at the calibration time, the calibration heat flux, a predetermined skin heat transfer coefficient, and the surface temperature of the object, which is obtained at the calibration time; and obtaining the heat loss coefficient between the core and the wrist based on a difference between the estimated calibration wrist temperature of the object and the core body temperature of the object which is obtained at the calibration time.

The method may further include, based on the obtained heat flux exceeding the predetermined reference value, outputting a text message for inducing a user to reduce the heat flux, to a display device.

According to a third aspect of the present invention, there is provided an electronic device including: a main body in which an apparatus for estimating body temperature is provided.

BRIEF DESCRIPTION OF THE DRAWINGS

The above and other objects and features of the present disclosure will become apparent by describing in detail example embodiments thereof with reference to the accompanying drawings:

FIG. 1 is a block diagram illustrating an apparatus for estimating body temperature according to an example embodiment;
FIG. 2 is a block diagram illustrating in detail a sensor according to an example embodiment;

FIGS. 3 and 4 are diagrams explaining an example of obtaining heat flux by using sensors and estimating wrist temperature of an object based on the heat flux according to an example embodiment;

FIGS. 5, 6A and 6B are diagrams explaining heat loss between a core and a center of a wrist;

FIG. 7 is a block diagram illustrating an apparatus for estimating body temperature according to an example embodiment;

FIG. 8 is a flowchart illustrating a method of estimating body temperature according to an example embodiment;

FIG. 9 is a flowchart illustrating a method of obtaining a heat loss coefficient between a core and a center of the wrist according to an example embodiment; and

FIGS. 10 to 15 are diagrams illustrating examples of structures of electronic devices including an apparatus for estimating body temperature according to an example embodiment.

DETAILED DESCRIPTION

Details of example embodiments are included in the following detailed description and drawings. Advantages and features of the disclosure, and a method of achieving the same will be more clearly understood from the following embodiments described in detail with reference to the accompanying drawings. Throughout the drawings and the detailed description, unless otherwise described, the same drawing reference numerals will be understood to refer to the same elements, features, and structures.

It will be understood that, although the terms first, second, etc. may be used herein to describe various elements, these elements should not be limited by these terms. These terms are only used to distinguish one element from another. Also, the singular forms are intended to include the plural forms as well, unless the context clearly indicates otherwise. It will be further understood that when an element is referred to as "comprising" another element, the element is intended not to exclude one or more other elements, but to further include one or more other elements, unless explicitly described to the contrary. In the following description, terms such as "unit" and "module" indicate a unit for processing at least one function or operation and they may be implemented by using hardware, software, or a combination thereof.

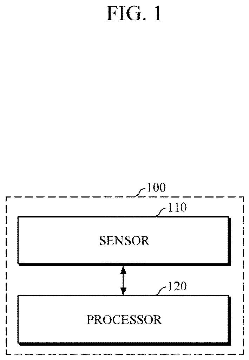

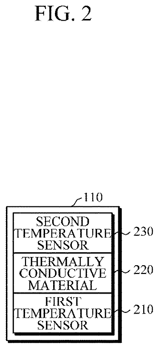

FIG. 1 is a block diagram illustrating an apparatus for estimating body temperature according to an embodiment of the present disclosure. FIG. 2 is a block diagram illustrating in detail a sensor according to an embodiment of the present disclosure.

Referring to FIG. 1, an apparatus 100 for estimating body temperature may include a sensor 110 and a processor 120.

The sensor 110 may include a plurality of sensors for obtaining data for estimating core body temperature from an object, and the processor 120 may estimate core body temperature of the object by using the data obtained from the plurality of sensors. The processor 120 may be electrically connected to the sensor 110, and in response to a request for estimating body temperature, the processor 120 may control the sensor 110.

Referring to FIG. 2, the sensor 110 may include a first temperature sensor 210 and a second temperature sensor 230. Further, the first temperature sensor 210 and the second temperature sensor 230 may be formed in a stacked structure with a thermally conductive material 220 being disposed therebetween.

The first temperature sensor 210 may be disposed at a lower end of the thermally conductive material 220 and measures surface temperature of the object; and the second temperature sensor 230 may be disposed at an upper end of the thermally conductive material 220 and measures surface temperature of the thermally conductive material 220. The thermally conductive material 220 may be, for example, an insulator having a size of 0.1 mm to 5 mm and may be a material (e.g., polyurethane foam) having a thermal conductivity of 0.1 W/mK or less. The size and thermal conductivity of the insulator are not limited thereto. Further, in an alternative amendment, an air-filled structure may also be provided in which air having a very low thermal conductivity is filled between the first temperature sensor 210 and the second temperature sensor 230, without using a separate material (e.g., the thermally conductive material 220) therebetween. The first temperature sensor 210 and the second temperature sensor 230 may include a thermistor. Among temperature sensors for measuring temperature, the thermistor is a contact type temperature sensor and may come into contact with, for example, a wrist of the object, to measure surface temperature of the wrist. Further, the first temperature sensor 210 and the second temperature sensor 230 may form a thermistor pair with the thermally conductive material 220 disposed therebetween.

The processor 120 estimates wrist temperature of the object based on the surface temperature of the object, which is measured by the first temperature sensor 210, and the surface temperature of the thermally conductive material 220 which is measured by the second temperature sensor 230, and estimates core body temperature of the object based on the estimated wrist temperature. Here, the term "wrist temperature" refers to temperature that is measured from a wrist, e.g., a center of a wrist.

For example, the processor 120 first obtains heat flux based on a value obtained by subtracting the surface temperature of the thermally conductive material 220, which is measured by the second temperature sensor 230, from the surface temperature of the object, which is measured by the first temperature sensor 210.

Then, the processor 120 estimates the wrist temperature of the object based on a resistance value of the thermally conductive material 220, the obtained heat flux, a predetermined skin heat transfer coefficient, and the surface temperature of the object.

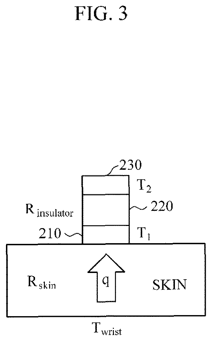

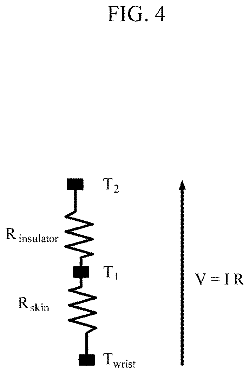

FIGS. 3 and 4 are diagrams explaining an example of obtaining heat flux by using sensors and estimating wrist temperature of an object based on the heat flux.

Referring to FIGS. 3 and 4, a difference between wrist temperature $T_{wrist}$ of the object and surface temperature $T_1$ of the object may be represented by the heat flux q. Assuming that heat transfer from the center of the wrist is formed by a series circuit, the same heat flux q may also be measured based on a temperature difference $T_1 - T_2$ between the surface temperature $T_1$ of the object, which is measured by the first temperature sensor 210, and the surface temperature $T_2$ of the thermally conductive material 220 which is measured by the second temperature sensor 220. In this case, regarding the heat transfer of the first temperature sensor 210 and the second temperature sensor 220, the following Equation 1 may be derived from Ohm's law (V = IR).

[Equation 1]

$$I = \frac{Twrist - T1}{Rskin} = \frac{T1 - T2}{Rinsulator}$$

Herein, $T_{wrist}$ denotes the wrist temperature of the object, $T_1$ denotes the surface temperature of the object, $T_2$ denotes the surface temperature of the thermally conductive material, $R_{skin}$ denotes the heat resistance, $R_{insulator}$ denotes the resistance of the thermally conductive material, and I denotes a thermoelectric current, in which I of Equation 1 may be represented by the following Equation 2.

[Equation 2]

$$\frac{Twrist - T1}{T1 - T2} = \frac{Rskin}{Rinsulator} = \frac{1}{\beta}$$

Herein, $\beta$ denotes the skin heat transfer coefficient. That is, the processor 120 may calculate the skin heat transfer coefficient $\beta$ based on the heat resistance $R_{insulator}$ of the thermally conductive material 220 and the heat flux $(T_1 - T_2)$. In this case, in order to estimate the wrist temperature of the object, a predetermined value may be used as the skin heat transfer coefficient $\beta$.

Equation 2 may be represented by the following Equation 3.

[Equation 3]

$$Twrist = T1 + \frac{T1 - T2}{\beta}$$

That is, the processor 120 may estimate the wrist temperature $T_{wrist}$ of the object by combining a ratio between the measured heat flux $(T_1 - T_2)$ and the skin heat transfer coefficient $\beta$ with the surface temperature $T_1$ of the object.

Then, the processor 120 estimates the core body temperature of the object based on the estimated wrist temperature $T_{wrist}$ of the object and the heat loss coefficient between the core and the center of the wrist. For example, the processor 120 may estimate the core body temperature of the object by linearly combining the estimated wrist temperature $T_{wrist}$ of the object with the heat loss coefficient between the core and the center of the wrist, which may be represented by the following Equation 4.

[Equation 4]

$$Tcore = Twrist + \alpha$$

Herein, $T_{core}$ denotes the core body temperature, and $\alpha$ denotes the heat loss coefficient between the core and the center of the wrist. While it is described that the heat loss coefficient is between the core and the center of the wrist, the disclosure is not limited thereto and the heat loss coefficient may be understood as between the core and any portion of the wrist.

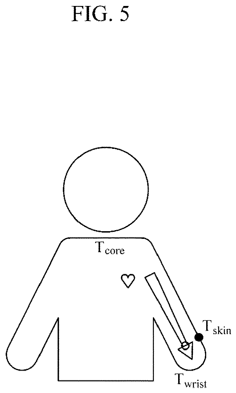

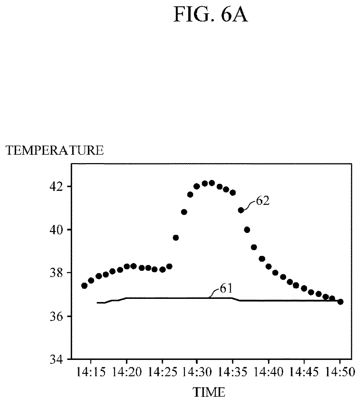

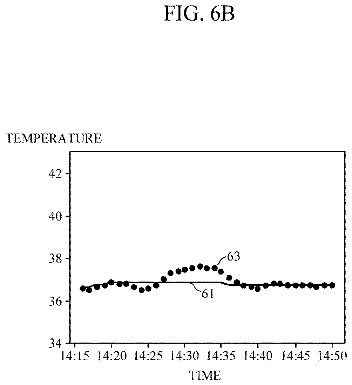

FIGS. 5, 6A and 6B are diagrams explaining heat loss between the core and the center of the wrist. The core body temperature may generally be defined as pulmonary artery temperature when blood is pumped out of the heart. In this case, it can be seen that heat loss occurs when the blood flows from the heart to the artery in the wrist. The heat loss, represented by a difference between the core body temperature $T_{core}$ and the wrist temperature $T_{wrist}$ may be referred to as the heat loss between the core and the center of the wrist.

When the core body temperature is estimated non-invasively based on the wrist skin temperature and the heat flux by using a simple conductive heat transfer model from the core to the skin ($T_{core} \rightarrow T_{skin}$), such heat loss is not reflected. However, according to an example embodiment, by reflecting the heat loss between the core and the wrist center using a two-stage heat transfer model of heat transfer from the core to the wrist center ($T_{core} \rightarrow T_{wrist}$) and heat transfer from the wrist center to the skin ($T_{wrist} \rightarrow T_{skin}$), the core body temperature may be estimated accurately. For example, FIG. 6A is a diagram illustrating a core body temperature 61 of the object which is measured over time, and an estimated core body temperature value 62 of the object which is obtained by using the simple conductive heat transfer model; and FIG. 6B is a diagram illustrating a core body temperature 61 of the object which is measured over time, and an estimated core body temperature value 63 of the object which is obtained by using the two-stage heat transfer model according to an example embodiment. In FIGS. 6A and 6B, during continuous measurement with a sensor worn on the object's wrist, a change in skin temperature and wrist temperature was induced by using a fan at a wind speed of 4.2 cm/sec. It can be seen that the estimated value 63 obtained by using the two-stage heat transfer model of FIG. 6B is closer to the core body temperature 61 than the estimated value 62 obtained by using the simple conductive heat transfer model of FIG. 6A.

According to another example embodiment of the present disclosure, when the wrist temperature is measured, a heat loss coefficient between the core and the wrist center is corrected, and the core body temperature may be estimated by using the corrected heat loss coefficient. For example, the processor 120 may correct the heat loss coefficient between the core and the wrist center based on external temperature at the time of estimating the core body temperature and external temperature at a calibration time.

The apparatus 100 may further include a temperature sensor (not shown), and the processor 120 may receive external temperature measured by the temperature sensor. Alternatively, the processor 120 may provide an interface through an output device, and may receive the external temperature at the time of estimating body temperature from a user through the interface.

Referring to the following Equation 5, the processor 120 may correct a temperature variation between the time of estimating the core body temperature and the calibration time by, for example, subtracting the external temperature at the time of estimating the core body temperature and the external temperature at the calibration time, and the processor 120 may accurately estimate the core body temperature by reflecting the temperature variation.

[Equation 5]

$$Tcore = Twrist + \alpha - \delta(Ta - Tb)$$

Herein, $\alpha$ denotes the heat loss coefficient between the core and the center of the wrist, Ta denotes the external temperature at the time of estimating the core body temperature, Tb denotes the external temperature at the calibration time, and $\delta$ denotes a predetermined coefficient.

By performing calibration at the calibration time, the processor 120 may obtain the heat loss coefficient between the core and the center of the wrist. Here, the calibration time may be a predetermined periodic time, a predetermined aperiodic time, a time before or after estimating the core body temperature, a time when an estimated value exceeds a threshold value, etc., but is not limited thereto.

For example, the processor 120 may obtain a calibration heat flux based on the surface temperature of the object and the surface temperature of the thermally conductive material which are obtained at the calibration time. If the obtained calibration heat flux is less than or equal to a predetermined reference value, the processor 120 may obtain the heat loss coefficient between the core and the center of the wrist.

For example, the processor 120 may first obtain the calibration heat flux by subtracting the surface temperature of the thermally conductive material which is obtained at the calibration time from the surface temperature of the object which is obtained at the calibration time.

If the obtained calibration heat flux is less than or equal to a predetermined reference value, the processor 120 may estimate a calibration wrist temperature of the object based on a resistance value of the thermally conductive material which is obtained at the calibration time, the calibration heat flux, a predetermined skin heat transfer coefficient, and the surface temperature of the object which is obtained at the calibration time. Here, the predetermined reference value may be, for example, a value of heat flux when a difference between the surface temperature of the object and the surface temperature of the thermally conductive material is 0.3 to 0.5, but is not limited thereto.

Then, the processor 120 may obtain the heat loss coefficient based on a difference between the estimated calibration wrist temperature of the object and the core body temperature of the object which is obtained at the calibration time. For example, the processor 120 may set, as the heat loss coefficient between the core and the wrist center, a value obtained by subtracting the calibration wrist temperature of the object from the core body temperature of the object which is obtained at the calibration time, or a value obtained by applying a predetermined weight to the resultant value, the predetermined weight being set for each user.

In this case, the processor 120 may obtain the calibration heat flux, the skin heat transfer coefficient, and the calibration wrist temperature by using the above Equations 1 to 3.

Here, the apparatus for estimating body temperature may further include a display device; and if the obtained calibration heat flux exceeds the reference value, the processor 120 may output a text message, inducing a user to reduce the heat flux, to the display device, so that the heat flux may be reduced to the reference heat flux or less. For example, the processor 120 may induce a user to reduce the heat flux by outputting a text message, such as "please cover your wrist, on which you wear your watch, with clothes or blankets for five minutes" or please cover your wrist, on which you wear your watch, with the hand on which the watch is not worn," and the like.

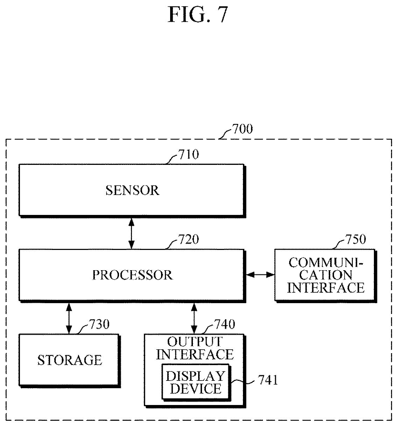

FIG. 7 is a block diagram illustrating an apparatus for estimating body temperature according to another example embodiment of the present disclosure.

Referring to FIG. 7, an apparatus 700 for estimating body temperature may include a sensor 710, a processor 720, a storage 730, an output interface 740, and a communication interface 750. In this case, the sensor 710 and the processor 720 may be the same as or similar to the sensor 110 and the processor 120 of FIG. 1, such that a detailed description thereof will be omitted.

The storage 730 may store information related to estimating core body temperature. For example, the storage 730 may store surface temperature of an object, surface temperature of a thermally conductive material, and processing results of the processor 920, such as heat flux, a heat transfer coefficient, and the like.

The storage 730 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The output interface 740 may provide the processing results of the processor 720 to a user. For example, the output interface 740 may display an estimated body temperature value of the processor 720 on a display. In this case, if the estimated body temperature value falls outside a normal range, the output interface 740 may provide a user with warning information by changing color, line thickness, etc., or displaying the abnormal value along with a normal range, so that the user may easily recognize the abnormal value. In addition, if the calibration heat flux, which is obtained during calibration, exceeds a reference heat flux, the output interface 740 may output a text message, inducing a user to reduce the heat flux, to the display device 741.

Further, along with or without the visual output, the output interface 740 may provide an estimated core body temperature value for the user in a non-visual manner by voice, vibrations, tactile sensation, and the like using an audio output module such as a speaker, or a haptic module and the like.

The display device 741 may include a display, a hologram device, or a projector and control circuitry to control the devices. The display device 741 may include touch circuitry adapted to detect a touch, and/or sensor circuitry (e.g., pressure sensor, etc.) adapted to measure the intensity of force incurred by the touch.

The audio module may convert a sound into an electrical signal or vice versa. The audio module may obtain the sound via the input device, or may output the sound via the sound output device, and/or a speaker and/or a headphone of another electronic device directly or wirelessly connected to the electronic device.

The haptic module may convert an electrical signal into a mechanical stimulus (e.g., vibration, motion, etc.) or electrical stimulus which may be recognized by a user by tactile sensation or kinesthetic sensation. The haptic module may include, for example, a motor, a piezoelectric element, and/or an electric stimulator.

The communication interface 750 may communicate with an external device to transmit and receive various data, related to estimating the core body temperature, to and from the external device. In this case, the external device may include an information processing device such as a smartphone, a tablet PC, a desktop computer, a laptop computer, and the like. For example, the communication interface 750 may transmit a body temperature estimation result to the external device, such as a user's smartphone and the like, so that the user may manage and monitor the estimation result by using a device having a relatively high performance.

The communication interface 750 may communicate with the external device by using various wired or wireless communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like. However, this is merely exemplary and is not intended to be limiting.

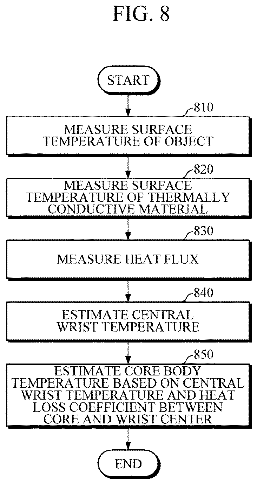

FIG. 8 is a flowchart illustrating a method of estimating body temperature according to an example embodiment of the present disclosure.

The method of FIG. 8 is an example of a method of estimating body temperature performed by the apparatuses 100 and 700 for estimating body temperature according to the embodiments of FIGS. 1 and 7, which are described above in detail, and thus will be briefly described below in order to avoid redundancy.

Referring to FIG. 8, the apparatus for estimating body temperature may first measures surface temperature of an object by using the first temperature sensor that may be disposed at a lower end of a thermally conductive material in 810.

Then, the apparatus for estimating body temperature measures surface temperature of the thermally conductive material by using the second temperature sensor that may be disposed at an upper end of the thermally conductive material in 820.

Subsequently, the apparatus for estimating body temperature measures heat flux based on the surface temperature of the object and the surface temperature of the thermally conductive material in 830. For example, the apparatus for estimating body temperature may measure the heat flux based on a value obtained by subtracting the surface temperature of the object and the surface temperature of the thermally conductive material.

Next, the apparatus for estimating body temperature estimates wrist temperature of the object based on the obtained heat flux and the surface temperature of the object in 840. For example, the apparatus for estimating body temperature may estimate the wrist temperature of the object by combining a resistance value of the thermally conductive material, the heat flux, a predetermined skin heat transfer coefficient, and the surface temperature of the object.

Then, the apparatus for estimating body temperature estimates core body temperature of the object based on the estimated wrist temperature of the object and a heat loss coefficient between the core and the wrist center in 850. For example, the apparatus for estimating body temperature may estimate the core body temperature of the object by linearly combining the estimated wrist temperature of the object and the heat loss coefficient between the core and the wrist center.

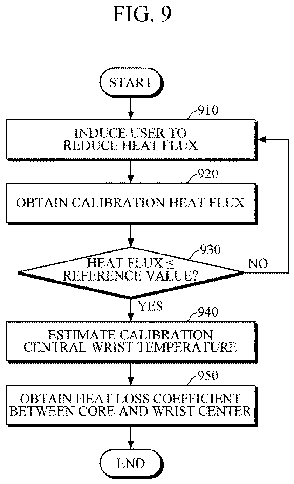

FIG. 9 is a flowchart illustrating a method of obtaining a heat loss coefficient between the core and the center of the wrist according to an example embodiment. The method of FIG. 9 is an example of a method of obtaining the heat loss coefficient between the core and the wrist center which is performed by the apparatuses 100 and 700 for estimating body temperature according to the embodiments of FIGS. 1 and 7, which are described in detail above, and thus will be briefly described below in order to avoid redundancy. Operations of FIG. 9 may be performed prior to the operations of FIG. 8.

Referring to FIG. 9, the apparatus for estimating body temperature may first induce a user to reduce heat flux through the display device in 910. For example, the apparatus for estimating body temperature may induce the user to reduce the heat flux by outputting a text message, such as "please cover your wrist, on which you wear your watch, with clothes or blankets for five minutes" or please cover your wrist, on which you wear your watch, with the hand on which the watch is not worn," and the like.

Then, the apparatus for estimating body temperature may obtain a calibration heat flux based on the surface temperature of the object and the surface temperature of the thermally conductive material, which are obtained at the calibration time, in 920. For example, the apparatus for estimating body temperature may obtain the calibration heat flux by subtracting the surface temperature of the thermally conductive material, which is obtained at the calibration time, from the surface temperature of the object, which is obtained at the calibration time.

Subsequently, if the obtained calibration heat flux is less than or equal to a predetermined reference value in 930, the apparatus for estimating body temperature may estimate a calibration wrist temperature of the object based on the obtained calibration heat flux, a predetermined skin heat transfer coefficient, and the surface temperature of the object which is obtained at the calibration time in 940. Here, the predetermined reference value may be, for example, a value of heat flux when a difference between the surface temperature of the object and the surface temperature of the thermally conductive material is 0.3 to 0.5, but is not limited thereto.

Then, the apparatus for estimating body temperature may obtain the heat loss coefficient between the core and the wrist center based on a difference between the estimated calibration wrist temperature of the object and the core body temperature of the object which is obtained at the calibration time in 950.

If the obtained heat flux exceeds the reference value in 930, the apparatus for estimating body temperature may reduce the heat flux to a value less than the reference value by repeating the operation 910 of inducing the user to reduce the heat flux. For example, the apparatus for estimating body temperature may output again the text message to induce the user to reduce the heat flux.

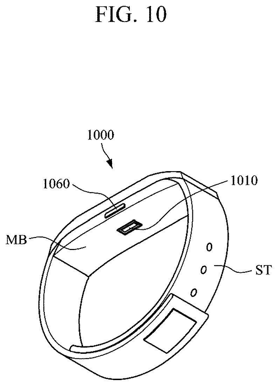

FIGS. 10 to 15 are diagrams illustrating examples of structures of electronic devices including the apparatuses 100 and 700 for estimating body temperature. Examples of the electronic device may include not only a smartphone, but also a smart watch, a smart band, smart glasses, a smart necklace, and an ear-wearable device, but the electronic device is not limited thereto.

Referring to FIG. 10, the electronic device may be implemented as a smart watch-type wearable device 1000, which includes a main body MB and a wrist strap ST.

The main body MB may be formed in various shapes, and a battery may be embedded in the main body MB and/or the strap ST to supply power to various components of the wearable device. The strap ST may be connected to both ends of the main body MB to allow the main body MB to be worn on a user's wrist, and may be flexible so as to be wrapped around the user's wrist. The strap ST may include a first strap and a second strap which are separated from each other. A first end of the first strap and a first end of the second strap may be connected to both sides of the main body MB, and a second end of the first strap and a second end of the second strap may be connected to each other via a fastening means. In this case, the connecting means may be formed as magnetic fastening, Velcro fastening, pin fastening, and the like, but is not limited thereto. Further, the strap ST is not limited thereto, and may be integrally formed as a non-detachable band.

The main body MB may include the apparatus for estimating body temperature. A sensor 1010, a processor, a display device, an output interface, a storage, and a communication interface may be mounted in the apparatus for estimating body temperature. However, depending on the size and shape of a form factor and the like, some of the display device, the output interface, the storage, and the communication interface may be omitted.

The sensor 1010 may include the first temperature sensor disposed at a lower end of the thermally conductive material configured to sense the surface temperature of the object, and the second temperature sensor disposed at an upper end of the thermally conductive material and configured to sense the surface temperature of the thermally conductive material. The sensor 1010 may be disposed on a rear surface of the main body MB, so that when worn on the user's wrist, the sensor 1010 may come into contact with an upper part of the wrist to obtain data for measuring core body temperature from the wrist.

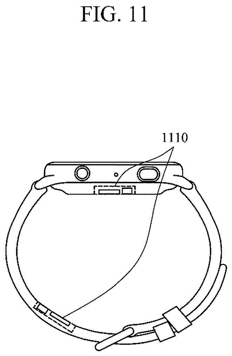

Referring to FIG. 11, a sensor 1110 may be disposed not only on the rear surface of the main body MB but also on the wrist strap ST to obtain data.

Referring back to FIG. 10, a manipulator 1060 may be formed on a side surface of the main body MB, as illustrated herein. The manipulator 1060 may receive a user's command and may transmit the received command to the processor. In addition, the manipulator 1060 may have a power button to turn on/off the wearable device 1000.

The processor mounted in the main body MB may be electrically connected to various components including the sensor 1010. The processor may estimate the core body temperature of the object by using data obtained from the plurality of sensors 1010. For example, the processor may obtain heat flux based on the surface temperature of the object and the surface temperature of the thermally conductive material, may estimate the wrist temperature of the object based on the obtained heat flux and the surface temperature of the object, and may estimate the core body temperature of the object based on the estimated wrist temperature of the object and the heat loss coefficient between the core and the wrist center. In addition, the processor may obtain a calibration heat flux based on the surface temperature of the object and the surface temperature of the thermally conductive material which are obtained at the calibration time; and if the obtained calibration heat flux is less than or equal to a reference value, the processor may obtain a heat loss coefficient between the core and the wrist center.

A display may be provided on a front surface of the main body MB and may display various application screens, including body temperature information, time information, received message information, and the like. For example, the display may display an estimated core body temperature value. In this case, if the estimated body temperature value falls outside a normal range, the processor may provide a user with warning information by changing color, line thickness, etc., or displaying the abnormal value along with a normal range, so that the user may easily recognize the abnormal value. Further, in response to a user's request, the processor may display not only the current estimated core body temperature value, but also continuous estimated core body temperature values over time and may provide the values to the user. In addition, the processor may display a variation in body temperature, e.g., a body temperature change during a day in graph form, and information as to whether the user has a deep sleep based on the body temperature change on the display. Furthermore, the processor may induce a user to reduce the heat flux. For example, the processor may induce the user to reduce the heat flux by outputting a text message, such as "please cover your wrist, on which you wear your watch, with clothes or blankets for five minutes" or "please cover your wrist, on which you wear your watch, with the hand on which the watch is not worn," and the like.

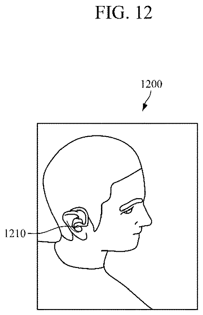

Referring to FIG. 12, the electronic device may be implemented as an ear-wearable device 1200.

The ear-wearable device 1200 may include a main body and an ear strap. A user may wear the ear-wearable device 1200 by hanging the ear strap on the user's auricle. The ear strap may be omitted depending on the shape of ear-wearable device 1200. The main body may be inserted into the external auditory meatus. A sensor device 1210 may be mounted in the main body. The ear-wearable device 1200 may provide the user with a component estimation result as sounds, or may transmit the estimation result to an external device, e.g., a mobile device, a tablet PC, a personal computer, etc., through a communication module provided in the main body.

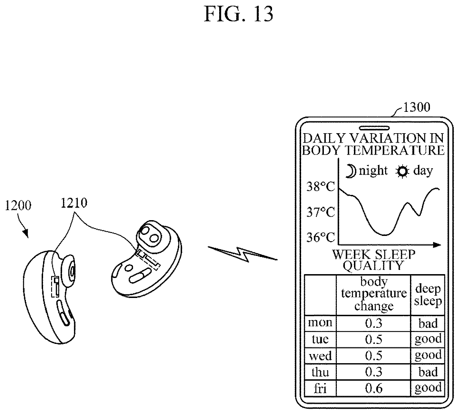

Referring to FIG. 13, the electronic device may be implemented by a combination of an ear-wearable device and a mobile device 1300 such as a smartphone. However, this is merely an example, and various other combinations of electronic devices may be provided. For example, a processor for estimating core body temperature may be mounted in a main body of the mobile device 1300. Upon receiving a request for estimating body temperature, the processor of the mobile device 1300 may communicate with a communication module mounted in the main body of the wearable device 1200, to obtain data by using the sensor 1210. Further, upon receiving data, such as the surface temperature of the object, the surface temperature of the thermally conductive material, etc., from the wearable device 1200, the processor may estimate core body temperature and may output an estimation result to the display of the mobile device 1300 through an output interface as illustrated herein.

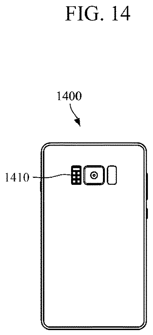

Referring to FIG. 14, the electronic device may be implemented as a mobile device 1400 such as a smartphone.

The mobile device 1400 may include a housing and a display panel. The housing may form an exterior of the mobile device 1400. The housing has a first surface, on which a display panel and a cover glass may be disposed sequentially, and the display panel may be exposed to the outside through the cover glass. A sensor 1410, a camera module and/or an infrared sensor, and the like may be disposed on a second surface of the housing.

For example, a plurality of sensors for obtaining data from a user may be disposed on a rear surface of the mobile device 1400, and a fingerprint sensor disposed on the front surface of the mobile device 1400, a power button or a volume button disposed on a side surface the mobile device 1400, a sensor disposed on other positions of the front and rear surfaces of the mobile device 1400, and the like may be provided to measure the core body temperature.

In addition, when a user transmits a request for estimating body temperature by executing an application and the like installed in the mobile device 1400, the mobile device 1400 may obtain data by using the sensor 1410, and may estimate the core body temperature and may provide the estimated value as images and/or sounds to the user by using the processor in the mobile device 1400.

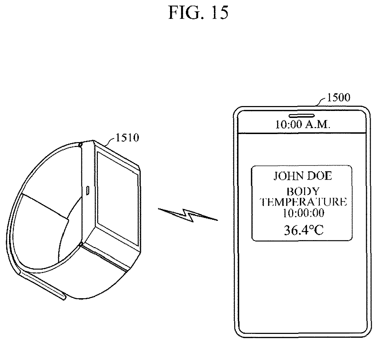

Referring to FIG. 15, the electronic device may be implemented as a combination of a wristwatch-type wearable device and a mobile device such as a smartphone. For example, a processor for estimating core body temperature may be mounted in a main body of the mobile device 1500. In response to receiving a request for measuring body temperature, the processor of the mobile device 1500 may communicate with a communication module, mounted in the main body of the wearable device 1510, to obtain data through the communication module. Further, upon receiving data, such as the surface temperature of the object, the surface temperature of the thermally conductive material, etc., from the wearable device 1510, the processor may estimate the core body temperature and may output an estimation result to a display of the mobile device 1500 through an output interface, as illustrated herein. In this case, in response to a user's request, the processor may display not only a current estimated core body temperature value, but also continuous estimated core body temperature values over time on a display to provide the values to the user. In addition, the processor may display a variation in body temperature, e.g., a body temperature change during a day in graph form, and information as to whether the user has a deep sleep based on the body temperature change on the display.

The disclosure may be realized as a computer-readable code written on a computer-readable recording medium. The computer-readable recording medium may be any type of recording device in which data is stored in a computer-readable manner.

Examples of the computer-readable recording medium include a ROM, a RAM, a CD-ROM, a magnetic tape, a floppy disc, an optical data storage, and a carrier wave (e.g., data transmission through the Internet). The computer-readable recording medium can be distributed over a plurality of computer systems connected to a network so that a computer-readable code is written thereto and executed therefrom in a decentralized manner. Functional programs, codes, and code segments needed for realizing the disclosure may be readily deduced by programmers of ordinary skill in the art to which the invention pertains.

At least one of the components, elements, modules or units (collectively "components" in this paragraph) represented by a block in the drawings may be embodied as various numbers of hardware, software and/or firmware structures that execute respective functions described above, according to an example embodiment. According to example embodiments, at least one of these components may use a direct circuit structure, such as a memory, a processor, a logic circuit, a look-up table, etc. that may execute the respective functions through controls of one or more microprocessors or other control apparatuses. Also, at least one of these components may be specifically embodied by a module,

10

a program, or a part of code, which contains one or more executable instructions for performing specified logic functions, and executed by one or more microprocessors or other control apparatuses. Further, at least one of these components may include or may be implemented by a processor such as a central processing unit (CPU) that performs the respective functions, a microprocessor, or the like. Two or more of these components may be combined into one single component which performs all operations or functions of the combined two or more components. Also, at least part of functions of at least one of these components may be performed by another of these components. Functional aspects of the above exemplary embodiments may be implemented in algorithms that execute on one or more processors. Furthermore, the components represented by a block or processing steps may employ any number of related art techniques for electronics configuration, signal processing and/or control, data processing and the like.

**Claims**

1. An apparatus for estimating body temperature, the apparatus comprising:

   a first temperature sensor (210) configured to measure (810) a surface temperature (T1) of an object;
   a second temperature sensor (230) configured to measure (820) a surface temperature (T2) of a material, which is positioned between the first temperature sensor (210) and the second temperature sensor (230); and
   a processor (120, 720) configured to obtain (830) a heat flux (T1-T2) based on the surface temperature (T1) of the object and the surface temperature (T2) of the material, configured to estimate (840) a wrist temperature (Twrist) of the object based on the obtained heat flux (T1-T2) and the surface temperature (T1) of the object, and configured to estimate (850) a core body temperature (Tcore) of the object based on the estimated wrist temperature (Twrist) of the object and a heat loss coefficient ($\alpha$) between a core and a wrist
   wherein the processor (120, 720) is further configured to obtain the heat flux (T1-T2) based on a value obtained by subtracting the surface temperature (T2) of the material from the surface temperature (T1) of the object, and to estimate the wrist temperature (Twrist) of the object based on a resistance value (Rinsulator) of the material, the heat flux (T1-T2), a predetermined skin heat transfer coefficient ($\beta$), and the surface temperature (T1) of the object.

2. The apparatus of claim 1, wherein the material comprises air.

3. The apparatus of claim 1 or 2, wherein at least one of the first temperature sensor (210) or the second temperature sensor (230) includes a thermistor.

4. The apparatus of one of claims 1 to 3, wherein the processor (120, 720) is further configured to estimate the core body temperature (Tcore) of the object by linearly combining the estimated wrist temperature (Twrist) of the object and the heat loss coefficient ($\alpha$) between the core and the wrist.

5. The apparatus of one of claims 1 to 4, wherein the processor (120, 720) is further configured to correct the heat loss coefficient ($\alpha$) between the core and the wrist based on an external temperature (Ta) at a time of estimating the core body temperature (Tcore) and an external temperature (Tb) at a calibration time.

6. The apparatus of one of claims 1 to 5, wherein the processor (120, 720) is further configured to obtain a calibration heat flux (T1 -T2) based on the surface temperature (T1) of the object and the surface temperature (T2) of the material, which are obtained at a calibration time, and the processor is further configured to, based on the obtained calibration heat flux (T1-T2) being less than or equal to a predetermined reference value, obtain the heat loss coefficient ($\alpha$) between the core and the wrist.

7. The apparatus of claim 6, wherein the processor (120, 720) is further configured to estimate a calibration wrist temperature (Twrist) of the object based on a resistance value (Rinsulator) of the material, which is obtained at the calibration time, the calibration heat flux (T1-T2), a predetermined skin heat transfer coefficient ($\beta$), and the surface temperature (T1) of the object which is obtained at the calibration time.

8. The apparatus of claim 7, wherein the processor (120, 720) is further configured to obtain the heat loss coefficient ($\alpha$) between the core and the wrist based on a difference between the estimated calibration wrist temperature (Twrist) of the object and the core body temperature (Tcore) of the object, which is obtained at the calibration time.

9. The apparatus of claim 6, further comprising a display device (741),

wherein the processor (120, 720) is further configured to, based on the obtained calibration heat flux (T1-T2) exceeding the predetermined reference value, output a text message for inducing a user to reduce the heat flux (T1-T2), to the display device (741).

10. A method of estimating body temperature, the method comprising:

measuring (810), by a first temperature sensor (210), a surface temperature (T1) of an object measuring (820), by a second temperature sensor (230), a surface temperature (T2) of a material disposed between the first temperature sensor (210) and the second temperature sensor (230);
obtaining (830) a heat flux (T1 -T2) based on the surface temperature (T1) of the object and the surface temperature (T2) of the material;
estimating (840) a wrist temperature (Twrist) of the object based on the obtained heat flux (T1-T2) and the surface temperature (T1) of the object; and
estimating (850) a core body temperature (Tcore) of the object based on the estimated wrist temperature (Twrist) of the object and a heat loss coefficient ($\alpha$) between a core and a wrist wherein the step of obtaining (830) the heat flux (T1-T2) comprises obtaining the heat flux (T1-T2) based on a value obtained by subtracting the surface temperature (T2) of the material from the surface temperature (T1) of the object, and
wherein the step of estimating (840) the wrist temperature (Twrist) of the object comprises estimating the wrist temperature (Twrist) based on a resistance value (Rinsulator) of the material, the heat flux, (T1-T2) a predetermined skin heat transfer coefficient ($\beta$), and the surface temperature (T1) of the object.

11. The method of claim 10 adapted to operate the apparatus for estimating body temperature according to one of claims 1 to 9.

12. An electronic device comprising:
a main body in which the apparatus for estimating body temperature according to one of claims 1 to 9 is provided.

**Patentansprüche**

1. Vorrichtung zum Schätzen der Körpertemperatur, wobei die Vorrichtung umfasst:

einen ersten Temperatursensor (210), konfiguriert zum Messen (810) einer Oberflächentemperatur (T1) eines Objekts;
einen zweiten Temperatursensor (230), konfiguriert zum Messen (820) einer Oberflächentemperatur (T2) eines Materials, das zwischen dem ersten Temperatursensor (210) und dem zweiten Temperatursensor (230) angeordnet ist; und
einen Prozessor (120, 720), konfiguriert zum Erhalten (830) eines Wärmestroms (T1-T2) basierend auf der Oberflächentemperatur (T1) des Objekts und der Oberflächentemperatur (T2) des Materials, konfiguriert zum Schätzen (840) einer Handgelenkstemperatur (Twrist) des Objekts basierend auf dem erhaltenen Wärmestrom (T1-T2) und der Oberflächentemperatur (T1) des Objekts, und konfiguriert zum Schätzen (850) einer Körperkerntemperatur (Tcore) des Objekts basierend auf der geschätzten Handgelenkstemperatur (Twrist) des Objekts und einem Wärmeverlustkoeffizienten ($\alpha$) zwischen einem Kern und einem Handgelenk,
wobei der Prozessor (120, 720) ferner konfiguriert ist zum Erhalten des Wärmestroms (T1-T2) basierend auf einem Wert, der durch Subtrahieren der Oberflächentemperatur (T2) des Materials von der Oberflächentemperatur (T1) des Objekts erhalten wird, und zum Schätzen der Handgelenkstemperatur (Twrist) des Objekts basierend auf einem Widerstandswert (Rinsulator) des Materials, dem Wärmestrom (T1-T2), einem vorbestimmten Hautwärmeübergangskoeffizienten ($\beta$) und der Oberflächentemperatur (T1) des Objekts.

2. Vorrichtung nach Anspruch 1, wobei das Material Luft umfasst.

3. Vorrichtung nach Anspruch 1 oder 2, wobei mindestens einer von dem ersten Temperatursensor (210) oder dem zweiten Temperatursensor (230) einen Thermistor enthält.

4. Vorrichtung nach einem der Ansprüche 1 bis 3, wobei der Prozessor (120, 720) ferner konfiguriert ist zum Schätzen der Körperkerntemperatur (Tcore) des Objekts durch lineare Kombination der geschätzten Handgelenkstemperatur (Twrist) des Objekts und des Wärmeverlustkoeffizienten ($\alpha$) zwischen dem Kern und dem Handgelenk.

5. Vorrichtung nach einem der Ansprüche 1 bis 4, wobei der Prozessor (120, 720) ferner konfiguriert ist zum Korrigieren des Wärmeverlustkoeffizienten ($\alpha$) zwischen dem Kern und dem Handgelenk basierend auf einer Außentemperatur (Ta) zu einem Zeitpunkt der Schätzung der Körperkerntemperatur (Tcore) und einer Außentemperatur (Tb) zu einem Kalibrierungszeitpunkt.

6. Vorrichtung nach einem der Ansprüche 1 bis 5, wobei der Prozessor (120, 720) ferner konfiguriert ist zum Erhalten eines Kalibrierungswärmestroms (T1-T2) basierend auf der Oberflächentemperatur (T1) des Objekts und der Oberflächentemperatur (T2) des Materials, die zu einer Kalibrierungszeit erhalten werden, und der Prozessor ferner konfiguriert ist, zum Erhalten des Wärmeverlustkoeffizienten ($\alpha$) zwischen dem Kern und dem Handgelenk basierend auf dem erhaltenen Kalibrierungswärmestrom (T1-T2), der kleiner als oder gleich einem vorbestimmten Referenzwert ist.

7. Vorrichtung nach Anspruch 6, wobei der Prozessor (120, 720) ferner konfiguriert ist zum Schätzen einer Kalibrierungshandgelenktemperatur (Twrist) des Objekts basierend auf einem Widerstandswert (Rinsulator) des Materials, der zum Kalibrierungszeitpunkt erhalten wird, dem Kalibrierungswärmestrom (T1-T2), einem vorbestimmten Hautwärmeübergangskoeffizienten ($\beta$) und der Oberflächentemperatur (T1) des Objekts, die zum Kalibrierungszeitpunkt erhalten wird.

8. Vorrichtung nach Anspruch 7, wobei der Prozessor (120, 720) ferner konfiguriert ist zum Erhalten des Wärmeverlustkoeffizienten ($\alpha$) zwischen dem Kern und dem Handgelenk basierend auf einer Differenz zwischen der geschätzten Kalibrierungshandgelenktemperatur (Twrist) des Objekts und der Körperkerntemperatur (Tcore) des Objekts, die zum Kalibrierungszeitpunkt erhalten wird.

9. Vorrichtung nach Anspruch 6, ferner umfassend eine Anzeigevorrichtung (741),
wobei der Prozessor (120, 720) ferner konfiguriert ist zum Ausgeben einer Textnachricht an die Anzeigevorrichtung (741) basierend auf dem erhaltenen Kalibrierungswärmestrom (T1-T2), der den vorbestimmten Referenzwert überschreitet, um einen Benutzer zu veranlassen, den Wärmestrom (T1-T2) zu reduzieren.

10. Verfahren zum Schätzen der Körpertemperatur, wobei das Verfahren umfasst:

Messen (810) einer Oberflächentemperatur (T1) eines Objekts durch einen ersten Temperatursensor (210);
Messen (820) einer Oberflächentemperatur (T2) eines Materials, das zwischen dem ersten Temperatursensor (210) und dem zweiten Temperatursensor (230) angeordnet ist, mit einem zweiten Temperatursensor (230);
Erhalten (830) eines Wärmestroms (T1-T2) basierend auf der Oberflächentemperatur (T1) des Objekts und der Oberflächentemperatur (T2) des Materials;
Schätzen (840) einer Handgelenkstemperatur (Twrist) des Objekts basierend auf dem erhaltenen Wärmestrom (T1-T2) und der Oberflächentemperatur (T1) des Objekts; und
Schätzen (850) einer Körperkerntemperatur (Tcore) des Objekts basierend auf der geschätzten Handgelenkstemperatur (Twrist) des Objekts und einem Wärmeverlustkoeffizienten ($\alpha$) zwischen einem Kern und einem Handgelenk,
wobei der Schritt des Erhaltens (830) des Wärmestroms (T1-T2) das Erhalten des Wärmestroms (T1-T2) basierend auf einem Wert umfasst, der durch Subtraktion der Oberflächentemperatur (T2) des Materials von der Oberflächentemperatur (T1) des Objekts erhalten wird, und
wobei der Schritt des Schätzens (840) der Handgelenktemperatur (Twrist) des Objekts das Schätzen der Handgelenktemperatur (Twrist) basierend auf einem Widerstandswert (Rinsulator) des Materials, dem Wärmestrom (T1-T2), einem vorbestimmten Hautwärmeübertragungskoeffizienten ($\beta$) und der Oberflächentemperatur (T1) des Objekts umfasst.

11. Verfahren nach Anspruch 10 angepasst zum Betreiben der Vorrichtung zum Schätzen der Körpertemperatur nach einem der Ansprüche 1 bis 9.

12. Elektronisches Gerät, umfassend:
einen Hauptkörper, in dem die Vorrichtung zum Schätzen der Körpertemperatur nach einem der Ansprüche 1 bis 9 vorgesehen ist.

**Revendications**

1. Appareil pour estimer la température corporelle, l'appareil comprenant :

   un premier capteur de température (210) configuré pour mesurer (810) une température de surface (T1) d'un objet ;
   un deuxième capteur de température (230) configuré pour mesurer (820) une température de surface (T2) d'un matériau, qui est positionné entre le premier capteur de température (210) et le deuxième capteur de température (230) ; et
   un processeur (120, 720) configuré pour obtenir (830) un flux de chaleur (T1-T2) basé sur la température de surface (T1) de l'objet et la température de surface (T2) 2 du matériau, configuré pour estimer (840) une température de poignet (Twrist) de l'objet basée sur le flux de chaleur obtenu (T1-T2) et la température de surface (T1) de l'objet, et configuré pour estimer (850) une température centrale du corps (Tcore) de l'objet sur la base de la température estimée du poignet (Twrist) de l'objet et d'un coefficient de perte de chaleur ($\alpha$) entre le coeur et un poignet,
   dans lequel le processeur (120, 720) est en outre configuré pour obtenir le flux de chaleur (T1-T2) sur la base d'une valeur obtenue en soustrayant la température de surface (T2) du matériau de la température de surface (T1) de l'objet, et pour estimer la température du poignet (Twrist) de l'objet sur la base d'une valeur de résistance (Rinsulator) du matériau, du flux de chaleur (T1-T2), d'un coefficient de transfert de chaleur cutané prédéterminé ($\beta$), et de la température de surface (T1) de l'objet.

2. Appareil selon la revendication 1, dans lequel le matériau comprend de l'air.

3. Appareil selon la revendication 1 ou 2, dans lequel au moins l'un du premier capteur de température (210) ou du deuxième capteur de température (230) inclut une thermistance.

4. Appareil selon l'une quelconque des revendications 1 à 3, dans lequel le processeur (120, 720) est en outre configuré pour estimer la température centrale du corps (tcore) de l'objet en combinant linéairement la température estimée du poignet (twrist) de l'objet et le coefficient de perte de chaleur ($\alpha$) entre le coeur et le poignet.

5. Appareil selon l'une quelconque des revendications 1 à 4, dans lequel le processeur (120, 720) est en outre configuré pour corriger le coefficient de perte de chaleur ($\alpha$) entre le coeur et le poignet sur la base d'une température externe (ta) au moment de l'estimation de la température corporelle (tcore) et d'une température externe (tb) au moment de l'étalonnage.

6. Appareil selon l'une quelconque des revendications 1 à 5, dans lequel le processeur (120, 720) est en outre configuré pour obtenir un flux de chaleur d'étalonnage (t1-t2) sur la base de la température de surface (t1) de l'objet et la température de surface (t2) du matériau, qui sont obtenues à un moment d'étalonnage, et
   le processeur est en outre configuré pour obtenir le coefficient de perte de chaleur ($\alpha$) entre le noyau et le poignet, sur la base du flux de chaleur d'étalonnage obtenu (T1-T2) qui est inférieur ou égal à une valeur de référence prédéterminée.

7. Appareil selon la revendication 6, dans lequel le processeur (120, 720) est en outre configuré pour estimer une température de poignet d'étalonnage (twrist) de l'objet sur la base d'une valeur de résistance (rinsulator) du matériau, obtenue au moment de l'étalonnage, du flux de chaleur d'étalonnage (t1-t2), d'un coefficient de transfert de chaleur cutané prédéterminé ($\beta$) et de la température de surface (t1) de l'objet obtenue au moment de l'étalonnage.

8. Appareil selon la revendication 7, dans lequel le processeur (120, 720) est en outre configuré pour obtenir le coefficient de perte de chaleur ($\alpha$) entre le coeur et le poignet sur la base d'une différence entre la température d'étalonnage estimée du poignet (twrist) de l'objet et la température corporelle (tcore) de l'objet, qui est obtenue au moment de l'étalonnage.

9. Appareil selon la revendication 6, comprenant en outre un dispositif d'affichage (741),
   dans lequel le processeur (120, 720) est en outre configuré pour, sur la base du flux de chaleur d'étalonnage obtenu (T1-T2) dépassant la valeur de référence prédéterminée, émettre un message textuel pour inciter l'utilisateur à réduire le flux de chaleur (T1-T2) sur le dispositif d'affichage (741).

10. Procédé d'estimation de la température corporelle, le procédé comprenant les étapes consistant à:

mesurer (810), par un premier capteur de température (210), une température de surface (T1) d'un objet mesurant (820), par un deuxième capteur de température (230), une température de surface (T2) d'un matériau disposé entre le premier capteur de température (210) et le deuxième capteur de température (230) ;

obtenir (830) un flux de chaleur (T1-T2) en fonction de la température de surface (T1) de l'objet et de la température de surface (T2) du matériau ;

estimer (840) la température du poignet (Twrist) de l'objet en fonction du flux de chaleur obtenu (T1-T2) et de la température de surface (T1) de l'objet ; et

estimer (850) une température corporelle (Tcore) de l'objet en fonction de la température estimée du poignet (Twrist) de l'objet et d'un coefficient de perte de chaleur ($\alpha$) entre le coeur et un poignet

dans lequel l'étape d'obtention (830) du flux de chaleur (T1-T2) comprend l'obtention du flux de chaleur (T1-T2) sur la base d'une valeur obtenue en soustrayant la température de surface (T2) du matériau de la température de surface (T1) de l'objet, et

dans lequel l'étape d'estimation (840) de la température du poignet (Twrist) de l'objet comprend l'estimation de la température du poignet (Twrist) sur la base d'une valeur de résistance (Rinsulator) du matériau, du flux de chaleur (T1-T2), d'un coefficient de transfert de chaleur cutané prédéterminé ($\beta$) et de la température de surface (T1) de l'objet.

11. Procédé selon la revendication 10 adaptée pour faire fonctionner l'appareil d'estimation de la température corporelle selon l'une des revendications 1 à 9.

12. Dispositif électronique comprenant :
un corps principal dans lequel se trouve l'appareil pour estimer la température corporelle selon l'une des revendications 1 à 9.

FIG. 1

FIG. 2

SECOND TEMPERATURE SENSOR ~ 230
THERMALLY CONDUCTIVE MATERIAL ~ 220
FIRST TEMPERATURE SENSOR ~ 210
110

FIG. 3

FIG. 4

$T_2$

$R_{insulator}$

$T_1$

$R_{skin}$

$T_{wrist}$

$V = I R$

FIG. 5

FIG. 6A

FIG. 6B

TEMPERATURE

FIG. 7

```
                                              ┌700
 ┌──────────────────────────────────────────────────────┐
 │                                    ┌710                │
 │  ┌──────────────────────────────────────┐             │
 │  │                                      │             │
 │  │              SENSOR                  │             │
 │  │                                      │             │
 │  └──────────────────────────────────────┘             │
 │                    ↕          ┌720                     │
 │  ┌──────────────────────────────────┐  ┌───────────┐ │
 │  │                                  │  │ COMMUNI-  │ │
 │  │           PROCESSOR              │↔ │  CATION   │ │
 │  │                                  │  │ INTERFACE │ │
 │  └──────────────────────────────────┘  └───────────┘ │
 │       ↕    ┌730        ↕   ┌740          ┌750        │
 │  ┌──────────────┐  ┌──────────────────┐             │
 │  │              │  │     OUTPUT       │             │
 │  │   STORAGE    │  │    INTERFACE     │             │
 │  │              │  │ ┌──────────┐     │             │
 │  │              │  │ │ DISPLAY  │ ┌741 │             │
 │  │              │  │ │ DEVICE   │     │             │
 │  └──────────────┘  └──────────────────┘             │
 └──────────────────────────────────────────────────────┘
```

FIG. 8

```
                        ┌─────────┐
                        │  START  │
                        └────┬────┘
                             │        ╭810
                  ┌──────────▼──────────┐
                  │   MEASURE SURFACE   │
                  │ TEMPERATURE OF OBJECT│
                  └──────────┬──────────┘
                             │        ╭820
                  ┌──────────▼──────────┐
                  │   MEASURE SURFACE   │
                  │ TEMPERATURE OF THERMALLY│
                  │ CONDUCTIVE MATERIAL │
                  └──────────┬──────────┘
                             │        ╭830
                  ┌──────────▼──────────┐
                  │  MEASURE HEAT FLUX  │
                  └──────────┬──────────┘
                             │        ╭840
                  ┌──────────▼──────────┐
                  │  ESTIMATE CENTRAL   │
                  │  WRIST TEMPERATURE  │
                  └──────────┬──────────┘
                             │        ╭850
                  ┌──────────▼──────────┐
                  │ ESTIMATE CORE BODY  │
                  │TEMPERATURE BASED ON CENTRAL│
                  │WRIST TEMPERATURE AND HEAT│
                  │LOSS COEFFICIENT BETWEEN│
                  │CORE AND WRIST CENTER│
                  └──────────┬──────────┘
                             │
                        ┌────▼────┐
                        │   END   │
                        └─────────┘
```

FIG. 9

```
                    ┌──────────┐
                    │  START   │
                    └────┬─────┘
                         │            ╱910
              ┌──────────▼──────────┐
              │    INDUCE USER TO   │◄────────┐
              │   REDUCE HEAT FLUX  │         │
              └──────────┬──────────┘         │
                         │          ╱920      │
              ┌──────────▼──────────┐         │
              │ OBTAIN CALIBRATION HEAT FLUX │ │
              └──────────┬──────────┘         │
                         │         ╱930       │
                       ╱─┴─╲                  │
                     ╱       ╲      NO         │
              ╱ HEAT FLUX ≤    ╲──────────────┘
              ╲ REFERENCE VALUE?╱
                ╲             ╱
                  ╲──┬──────╱
                     │ YES
                     │         ╱940
              ┌──────▼──────────────┐
              │  ESTIMATE CALIBRATION │
              │ CENTRAL WRIST TEMPERATURE │
              └──────────┬──────────┘
                         │         ╱950
              ┌──────────▼──────────┐
              │ OBTAIN HEAT LOSS COEFFICIENT │
              │ BETWEEN CORE AND WRIST CENTER │
              └──────────┬──────────┘
                         │
                    ┌────▼─────┐
                    │   END    │
                    └──────────┘
```

FIG. 10

FIG. 11

FIG. 12

FIG. 13

FIG. 14

FIG. 15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019142280 A1
- WO 2021057873 A1